# EUROPEAN PATENT APPLICATION

(11) **EP 4 246 524 A1**
(43) Date of publication of application: **20.09.2023**
(21) Application number: 22203816.8
(22) Date of filing: 26.10.2022
(51) Int. Cl.: G16H 20/40, A41D 13/12, G16H 40/20, A61B 5/00, A41D 1/00, A61B 90/90, A61B 90/98, G06Q 10/0833

(54) **SURGICAL GOWN TRACKING SYSTEM AND SURGICAL GOWN THEREOF**

(30) Priority: 18.03.2022 TW 111202749 U
(71) Applicant: AI Bioelectronic Healthtech Co., Ltd., 32450 Taoyuan City (TW)
(72) Inventor: HO, Yen-Yi, 32450 Taoyuan City (TW); HUANG, Yen-Yun, 32450 Taoyuan City (TW)
(74) Representative: Viering, Jentschura & Partner mbB Patent- und Rechtsanwälte

(57) **Abstract**

A surgical gown tracking system (900) is used for tracking a surgical gown (1). The surgical gown tracking system (900) includes a chip (20) and a chip reader (100). The chip (20) is located on the surgical gown (1), and the chip (20) is used for recording a usage record of the surgical gown (1) and managing inventory. The chip reader (100) is electrically connected to the chip (20) and used for reading the usage record of the surgical gown (1).

## Description

### CROSS REFERENCE TO RELATED APPLICATIONS

This application claims priority of Application No. 111202749 filed in Taiwan on March 18, 2022 under 35 U.S.C. § 119, the entire contents of all of which are hereby incorporated by reference.

### BACKGROUND OF THE INVENTION

### 1. Field of the Invention

The present invention relates to a surgical gown tracking system and a surgical gown thereof; more particularly, the present invention relates to a surgical gown tracking system and a surgical gown thereof for tracking and managing the surgical gown conveniently.

### 2. Description of the Related Art

The surgical gown is a garment that medical staff must wear during surgery. The surgical gown is used for isolating the bodily fluids of the patient while the surgery is performed by the medical staff, to prevent the medical staff from touching the bodily fluids and becoming infected, and to prevent bodily fluids from contaminating the clothes worn under the surgical gown by medical staff. The common surgical gown is made of materials that are easy to clean, convenient to replace, waterproof and comfortable. However, the surgical gown has an upper limit of the usage amount; when the surgical gown reaches the upper limit of the usage amount, the water resistance will gradually decrease, and the surgical gown, having exceeded the upper limit of the usage amount, must be discarded.

However, the number of surgical gowns that the hospital has to manage is enormous, so it is difficult to record the total usage and usage frequency of each surgical gown in detail. Therefore, there is a need for a system for conveniently tracking and managing surgical gowns.

### SUMMARY OF THE INVENTION

It is an object of the present invention to provide a surgical gown tracking system which allows convenient tracking and management of the surgical gown.

To achieve the abovementioned object, a surgical gown tracking system of the present invention is used for tracking a surgical gown, and the surgical gown tracking system includes a chip and a chip reader. The chip is located on the surgical gown, and the chip is used for recording a usage record of the surgical gown. The chip reader is electrically connected to the chip for reading the usage record of the surgical gown.

According to one embodiment of the present invention, the surgical gown tracking system further includes a server, the server is electrically connected to the chip reader, and the chip reader uploads the usage record to the server.

According to one embodiment of the present invention, the usage record includes a medical staff behavior data, a surgical gown user data and a surgical gown data.

Another object of the present invention is to provide a surgical gown which is convenient to track and manage.

To achieve the abovementioned object, a surgical gown of the present invention is applied to a surgical gown tracking system, and the surgical gown tracking system includes a chip reader and a chip. The surgical gown includes a garment main body. The chip is located on the garment main body and electrically connected to the chip reader, and the chip is used for recording a usage record of the surgical gown, which is read by the chip reader.

According to one embodiment of the present invention, the surgical gown further includes a protective layer, and the protective layer covers the chip.

According to one embodiment of the present invention, the protective layer is made of silicone.

### BRIEF DESCRIPTION OF THE DRAWINGS

FIG. 1 presents a schematic drawing of the surgical gown in one embodiment of the present invention.
FIG. 2 presents a system structure drawing of the surgical gown tracking system in one embodiment of the present invention.

### DETAILED DESCRIPTION OF THE PREFERRED EMBODIMENT

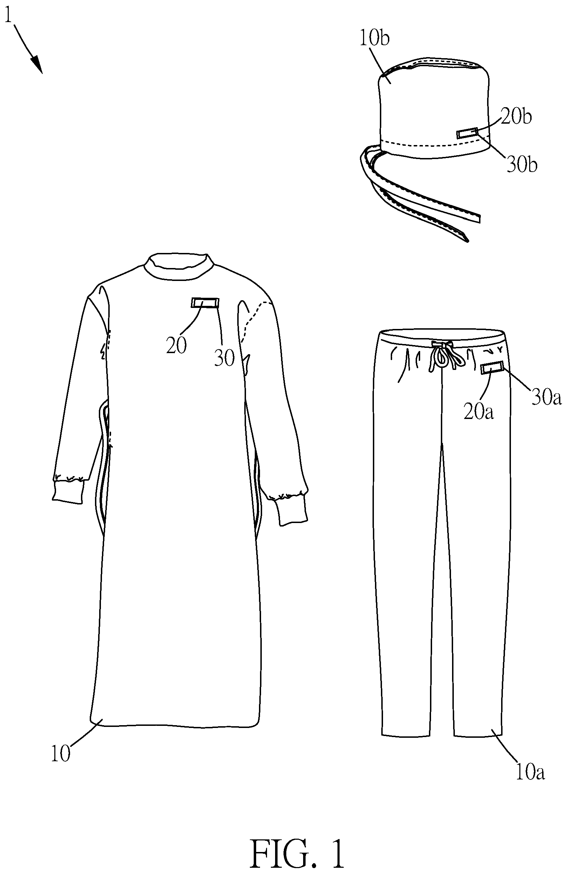

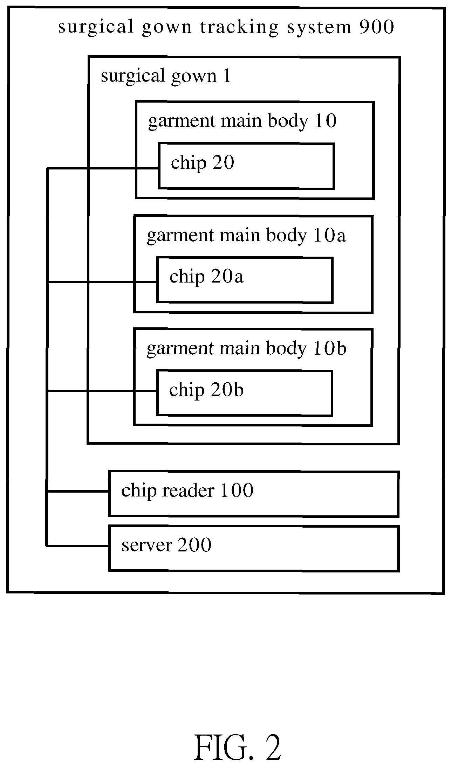

Please refer to FIG. 1 to FIG. 2, which illustrate the surgical gown tracking system and the surgical gown in one embodiment of the present invention. FIG. 1 presents a schematic drawing of the surgical gown in one embodiment of the present invention. FIG. 2 presents a system structure drawing of the surgical gown tracking system in one embodiment of the present invention.

As shown in FIG. 1 and FIG. 2, in one embodiment of the present invention, the surgical gown tracking system 900 is used for allowing the medical staff to conveniently count, record and manage the surgical gowns used in the hospital. The surgical gown tracking system 900 includes three chips 20, 20a, 20b, a chip reader 100 and a server 200.

In one embodiment of the present invention, the surgical gown 1 includes three garment main bodies 10, 10a, 10b and three protective layers 30, 30a, 30b. The garment main bodies 10, 10a, 10b are respectively the top, the trousers and the hat; however, the types of the garment main bodies 10, 10a, 10b are not limited to the abovementioned description, and the type can also be other garments, such as a glove or a mask. The amounts of the surgical gown 1 and the garment main bodies 10, 10a, 10b are not limited to the abovementioned description, and the amount can be changed to be more than one according to user requirements.

In one embodiment of the present invention, the three chips 20, 20a, 20b are radio frequency identification (RFID) chips, which are respectively located on the outside of the three garment main bodies 10, 10a, 10b. The three chips 20, 20a, 20b are used for recording a usage record of the surgical gown 1. The usage record includes a medical staff behavior data, a surgical gown user data and a surgical gown data.

The medical staff behavior data, for example, are the surgery starting times and surgery ending times of the medical staff. The surgical gown data are the departments (such as internal medicine, orthopedics, hematology oncology, etc.), the titles (such as attending physician, resident, anesthesiologist, nurse, etc.) and the names of the medical staff. The surgical gown data are the size of the surgical gown 1, the current usage amount and the upper limit of the usage amount of the surgical gown 1. However, the amount of the chips 20, 20a, 20b is not limited to the abovementioned design, for the amount of the chips 20, 20a, 20b can be changed according to the amount of the garment main bodies 10, 10a, 10b.

In one embodiment of the present invention, the three protective layers 30, 30a, 30b respectively cover the three chips 20, 20a, 20b. The protective layers 30, 30a, 30b are made of medical grade silicone, the protective layers 30, 30a, 30b are used for preventing the three chips 20, 20a, 20b from being damaged by contact with external dust or liquids, and the protective layers 30, 30a, 30b have high corrosion resistance and high temperature resistance (the applicable temperature range of the protective layers 30, 30a, 30b of the present invention is -40-200°C); therefore, when the user wants to wash the surgical gown 1, the user can directly put the surgical gown 1 into a washing machine for dry cleaning and drying, and the protective layers 30, 30a, 30b can prevent the chips 20, 20a, 20b from being damaged by contact with the cleaning solution or by temperature. However, the amount of the protective layers 30, 30a, 30b is not limited to the abovementioned design, and the amount of the protective layers 30, 30a, 30b can be changed according to the amount of the chips 20, 20a, 20b.

In one embodiment of the present invention, the chip reader 100 is, for example, a radio frequency identification (RFID) reader or a near-field communication (NFC) tag reader. The chip reader 100 can be installed in various hospitals or manufacturing plants of the surgical gown 1, and the chip reader 100 is electrically connected to the chips 20, 20a, 20b and an external computer 300 of the hospital. The user can operate the chip reader 100 to read the usage records of the surgical gown 1 recorded in the chips 20, 20a, 20b, and the user can also operate the chip reader 100 and the external computer 300 to write information into the chips 20, 20a, 20b. The server 200 is set up on the network and electrically connected to the chip reader 100, and the chip reader 100 can upload the usage records which are obtained from the chips 20, 20a, 20b to the server 200.

After the medical supplies manufacturer produces the surgical gown 1, the manufacturer can use the chip reader 100 to electrically connect to the chips 20, 20a, 20b and operate the external computer 300 to enter the size of the surgical gown 1, the current usage amount and the upper limit of the usage amount to each of the chips 20, 20a, 20b via the electrical connection between the chip reader 100 and the chips 20, 20a, 20b such that the medical staff can count, track and manage the surgical gown 1. When the surgical gown 1 is sent to the hospital warehouse, the warehouse staff can use the chip reader 100 of the hospital to read the size of the surgical gown 1, the current usage amount and the upper limit of the usage amount saved in the chips 20, 20a, 20b and then upload the current usage amount and the upper limit of the usage amount and the size of the surgical gown 1 to the server 200 via the electrical connection between the chip reader 100 of the hospital and the external computer 300.

Then, if a medical staff member needs wear the surgical gown 1 while treating a patient, the medical staff member can go to the warehouse to retrieve the surgical gown 1 and use the chip reader 100 and the external computer 300 of the hospital to enter the department and the title of the medical staff member into the chips 20, 20a, 20b and update the current usage amount of the surgical gown 1.

Then, before the medical staff member dons the surgical gown 1 to prepare to perform the surgery, the medical staff member can use the chip reader 100 and the external computer 300 of the hospital to enter the surgery starting time into the chips 20, 20a, 20b. After the medical staff member finishes the surgery, the medical staff member can use the chip reader 100 and the external computer 300 of the hospital to enter the surgery ending time into the chips 20, 20a, 20b.

Finally, when the medical staff member finishes the surgery and returns the surgical gown 1 to the warehouse, the warehouse staff can use the chip reader 100 of the hospital to read the chips 20, 20a, 20b and to upload the department and title of the medical staff member, the surgery starting time and the surgery ending time, and the current usage amount of the surgical gown 1 saved in the chips 20, 20a, 20b to the server 200 via the external computer 300. Subsequently, the server 200 can store the information, and the warehouse staff can query and track the usage record of the surgical gown 1 to check and confirm whether the surgical gown 1 has been returned to storage. Furthermore, when the current usage amount of the surgical gown 1 reaches the upper limit of the usage amount, the server 200 can transmit a message to the external computer 300 to notify the warehouse staff that the surgical gown 1 must be discarded.

Via the design of the surgical gown tracking system 900 and the surgical gown 1 of the present invention, the medical staff can conveniently count, record and manage the usage of surgical gowns in the hospital; via the protection of the protective layer for the chip, the surgical gown 1 can be washed in an industrial washing machine without the chip being damaged by the dry cleaning and drying processes.

## Claims

1. A surgical gown tracking system (900), for tracking a surgical gown (1), the surgical gown tracking system (900) comprising:
a chip (20), located on the surgical gown (1), wherein the chip (20) is used for recording a usage record of the surgical gown (1); and
a chip reader (100), electrically connected to the chip (20), for reading the usage record of the surgical gown (1).

2. The surgical gown tracking system (900) as claimed in Claim 1, further comprising a server (200), wherein the server (200) is electrically connected to the chip reader (100) and the chip reader (100) uploads the usage record to the server (200).

3. The surgical gown tracking system (900) as claimed in Claim 2, wherein the usage record comprises a medical staff behavior data, a surgical gown user data and a surgical gown data.

4. A surgical gown (1), applied to a surgical gown tracking system (900), wherein the surgical gown tracking system (900) comprises a chip reader (100) and a chip (20), the surgical gown (1) comprising:
a garment main body (10);
wherein the chip (20) is located on the garment main body (10) and electrically connected to the chip reader (100), and the chip (20) is used for recording a usage record of the surgical gown (1), allowing the chip reader (100) to read the usage record of the surgical gown (1).

5. The surgical gown (1) as claimed in Claim 4, further comprising a protective layer (30), and the protective layer (30) covers the chip (20).

6. The surgical gown (1) as claimed in Claim 5, wherein the usage record comprises a medical staff behavior data, a surgical gown user data and a surgical gown data.

7. The surgical gown (1) as claimed in Claim 6, wherein the protective layer (30) is made of silicone.
